# EUROPÄISCHE PATENTSCHRIFT

(11) **EP 2 444 237 B1**
(45) Veröffentlichungstag und Bekanntmachung des Hinweises auf die Patenterteilung: **06.06.2018**
(21) Anmeldenummer: 12151232.1
(22) Anmeldetag: 05.04.2007
(51) Int. Cl.: B29C 65/16, A61M 39/10

(54) **Durchstrahllaserschweißverfahren für die Verbindung von Kunststoffformkörpern und Fügeverbindung**
Transmission laser welding method for joining plastic moulded articles and welded joint
Procédé de soudage laser par transmission pour la connexion de corps moulés en matière plastique et joint soudé

(30) Priorität: 06.04.2006 DE 102006016299
(43) Veröffentlichungstag der Anmeldung: 25.04.2012
(62) Teilanmeldung aus: 07724068.7
(73) Patentinhaber: Fresenius Medical Care Deutschland GmbH, 61352 Bad Homburg (DE)
(72) Erfinder: Kreischer, Thomas, 66113 Saarbrücken (DE); Kugelmann, Franz, 66606 St.Wendel/Bliesen (DE)
(74) Vertreter: Stolmár & Partner Patentanwälte PartG mbB

(56) Entgegenhaltungen:
- WO-A-2005/097471
- WO-A-2005/113049
- DE-A1-102005 010 193
- JP-A- 1 151 464
- JP-A- 2005 104 132
- JP-A- 2005 305 985
- US-A1- 2006 086 701

## Beschreibung

Die vorliegende Erfindung betrifft ein Durchstrahllaserschweißverfahren zum Verschweißen von insbesondere Schlauchsegmenten aus Kunststoff mit im Wesentlichen röhrenförmigen Formkörpern wie weitere Schlauchsegmente, Konnektoren, Fittings, Verschlüssen etc.

Zur formschlüssigen Verbindung von Formkörpern aus Kunststoffen, insbesondere aus verschiedenen Kunststoffen, existieren grundsätzlich verschiedene Möglichkeiten. Beispielsweise können die zwei Formkörper mittels Klebe- oder Schweißverfahren miteinander formschlüssig verbunden werden.

Unter den Schweißverfahren wird neben IR-Schweißen (siehe z.B. WO 2005/080067) das Schweißen mittels Laserstrahlung in letzter Zeit besonders intensiv untersucht.

Das grundlegende physikalische Prinzip eines Schweißverfahrens mittels Laser erfordert, daß mindestens ein Teil des eingesetzten Laserlichts von dem Material mindestens eines der beiden miteinander zu verbindenden Formteile in zumindest so einem Ausmaß absorbiert wird, daß es in Wärme umgewandelt werden kann und das Material an der durch das Laserlicht erwärmten Stelle durch Erwärmung fließfähig wird und eine schlüssige Verbindung mit dem Kunststoffmaterial des zweiten Formteils ermöglicht.

Das Schweißverfahren begrenzende Parameter sind die Wellenlänge des verwendeten Laserlichts und das Absorptionsverhalten des Kunststoffes bei dieser Wellenlänge. Insbesondere werden dabei Hochleistungsdiodenlaser mit einer Wellenlänge von 610-840 Nanometern und Nd:YAG-Festkörperlaser mit einer Wellenlänge von circa 1.050 Nanometern entweder im Bereich des sichtbaren Lichts (400-750 Nanometer) bzw. ein im infraroten Bereich verwendet. Es können allerdings auch oftmals CO₂-Gaslaser mit einer Wellenlänge von circa 11.000 Nanometern verwendet werden.

Die Absorptionseigenschaften und damit die Bearbeitung von Kunststoffen sind in Abhängigkeit von der verwendeten Wellenlänge der Laserstrahlung sehr unterschiedlich. Inhomogenitäten im Kunststoff, wie beispielsweise Pigmente, Füll- oder Verstärkungsstoffe, aber auch kristalline Überstrukturen bei teilkristallierenden Kunststoffen streuen die eingekoppelte Strahlung und setzen insbesondere die Eindringtiefe der Strahlung in den Kunststoff herab.

Trifft ein Laserstrahl auf ein zu erwärmendes Kunststoffteil, so wird die Laserstrahlung zu verschiedenen Teilen reflektiert, absorbiert und transmittiert. Die Intensitätsabnahme der in den Kunststoff eindringenden Strahlung lässt sich in Abhängigkeit von der Materialtiefe nach dem so genannten Bougourschen Gesetz beschreiben. Dabei nimmt die eingekoppelte Intensität exponentiell mit der Materialtiefe ab.

Probleme treten insbesondere durch die thermische Zersetzung durch Strahlungserwärmung von Kunststoffen, insbesondere mittels CO₂-Lasern auf. Dies liegt an der schlechten Wärmeleitfähigkeit von Kunststoffen, da die Oberflächentemperatur des Kunststoffteils oftmals schnell ansteigt, wobei die Gefahr einer thermischen Materialzersetzung besteht. [Die Grundlagen von Laserschweißverfahren sind beispielsweise bei H. Potente et al. "Laserschweißen von Thermoplasten" (Plastverarbeiter 1995, Nr. 9, S. 42 ff.), F. Becker et al. "Trends bei Serienschweißverfahren in Kunststoffe 87 (1997, S. 11 ff.) sowie von H. Puetz et al. in Modern Plastics, (1997, S. 121 ff.) dargestellt.]

Das Absorptionsverhalten und damit auch die Transmission von einem bei einer bestimmten Wellenlänge lasertransparenten Polymer bzw. Kunststoff kann z.B. durch das Zumischen von Absorbern gesteuert werden. Derartige Absorber sind beispielsweise Ruß bzw. auch spezielle Farbstoffe, die in den letzten Jahren entwickelt wurden.

Eine Reihe von Farbstoffen, die ein derartiges steuerbares Absorptionsverhalten ermöglichen, sind kommerziell erhältlich und wurden speziell dafür entwickelt, in Polymermischungen zugemischt zu werden, um eine Laserverschweißung bei definierten Wellenlängen zu ermöglichen. Dafür bieten sich auch die bei I.A. Jones et al. "Use of infrared dyes for Transmission Laser Welding of Plastics" (Tech 2000 Conference Proceedings, S. 1166 ff.) offenbarten Farbstoffe an.

Eine spezielle Form des Laserschweißverfahrens, nämlich Laserdurchstrahlschweißverfahren hat gegenüber anderen Schweißverfahren auch den Vorteil, daß auch komplexe Geometrien der Fügefläche schnell und rationell verschweißt werden können. Bei der Verschweißung von Schlauchstücken mit z. B. Konnektoren, Fittings etc. ist es nötig, daß ein Fügepartner vom Laserstrahl vollständig durchstrahlt wird, d. h. also Laserstrahlung nicht absorbiert. Der zweite bzw. Teile des zweiten Fügepartners müssen das Laserlicht unter Hitzeentwicklung absorbieren können. Üblicherweise erfolgt die Bestrahlung von der Außenseite eines Fügepartners aus.

Die WO 2005/113049 A betrifft ein Verfahren zum Laserschweißen von röhrenförmigen polymeren Katheterkomponenten, bei dem die Katheterkomponenten mit einem Lasersystem durch eine Wärmefusionsverbindung verbunden werden. Das Lasersystem kann eine einzelne Wellenlänge aufweisen, die eine hohe Absorption im Material einer inneren Katheterkomponente oder in den Materialien aller Katheterkomponenten besitzt, bzw. in einer Farbe, mit der die Katheterkomponenten versehen sein können.

Die WO 2005/097471 A beschreibt ein Verfahren zum Laserschweißen von röhrenförmigen polymeren Katheterkomponenten, wobei eine erste Komponente eine relativ geringe Absorption für die Laserenergie und die zweite Komponente, die eine Farbe aufweisen kann, eine relativ hohe Absorption für die Laserenergie besitzt. Dabei dringt der Laserstrahl durch die erste Katheterkomponente ohne nennenswerte Erzeugung von Wärme. Wärme zum Bilden einer Fusionsbindung wird erzeugt, wenn der Laserstrahl auf den Kontaktbereich zwischen der ersten und zweiten Katheterkomponente trifft.

Die US 2006/0086701 A1 betrifft ein Verfahren zum Schweißverbinden von röhrenförmigen polymeren Komponenten medizinischer Vorrichtungen, bei dem ein kollimierter oder pseudokollimierter Energiestrahl auf die zu verschweißenden Stellen der entsprechenden Komponenten gerichtet wird.

Die DE 10 2005 010 193 A1 betrifft ein Verfahren zum Verbinden sich in einem Fügeabschnitt überlappender Kunststoffrohre, bei dem der Fügeabschnitt mit nicht kohärenter Strahlung bestrahlt wird, wodurch sich nach dem Aufschmelzen der Rohre die gewünschte Verbindung ausbildet. Dabei kann das außenliegende Rohr für die Strahlung transparent sein und nur das innenliegende Rohr absorbiert die Strahlung.

Die JP 1 151464 A betrifft ein Verfahren zum Schweißverbinden röhrenförmiger Komponenten medizinischer Vorrichtungen, die jeweils aus einem aus Vinylchlorid-Harz bestehen, wobei die eine Komponente ein T-förmiges Formteil und die andere Komponente ein Rohr darstellt, und wobei das Rohr vor dem Bestrahlen in das Formteil gesteckt wird.

Die JP 2005 104132 A beschreibt ein Verfahren zum Laserschweißen für ein röhrenförmiges Segment mit einer inneren Lage und einer äußeren Lage bestehend aus einem fluorhaltigen Kunststoff. Vor dem Schweißvorgang werden die Oberflächen der beiden zu verschweißenden Materialien aufgeraut und anschließend eine Schicht eines Laserstrahl-absorbierenden Materials zwischen den beiden Schichten angebracht, die sich durch Lasereinstrahlung erwärmt.

Die JP 2005 305985 A betrifft ein Verfahren zum Laserschweißen zweier röhrenförmiger Komponenten, die jeweils aus einem für die Strahlung transparenten polymeren Material bestehen. Dabei wird auf die Außenfläche der Endsegmente der zu verbindenden Komponenten eine Schicht eines Laserstrahl-absorbierenden Materials aufgebracht, die Endsegmente in ein Anschlussstück eingebracht und das absorbierende Material mittels Lasereinstrahlung ausgehärtet.

Die EP 1552916 A1 beschreibt beispielsweise das Verbinden von röhrenförmigen Formkörpern mittels Laserschweißverfahren, wobei jedoch der Laser um die zu fügende Verbindung der Formkörper gedreht werden muss, was eine aufwendige Apparatur und Mechanik erfordert, damit zwei dreidimensionale Teile miteinander fugenlos verschweißt werden können.

Die US 2003/0141634 beschreibt laserverschweißbare Schläuche für medizinische Anwendungen, wobei in einem mehrschichtig aufgebauten Schlauch jede Schicht ein laserabsorbierendes Material umfasst. Auch hier ist es notwendig, daß entweder der Schlauch oder die gesamte Laserdurchstrahlapparatur einer Drehbewegung unterzogen werden muss, damit die Schläuche über ihren gesamten Umfang miteinander verschweißt werden können.

Die DE 10245355 A1 beschreibt die Verbindung eines Rohrelementes über eine Manschette. In einem so genannten Laserdurchstrahlverfahren werden ein Rohrabschnitt, ein so genannter Konnektor, und die Manschette miteinander verschweißt. Das Verschweißen erfordert hier ebenfalls eine um den gesamten Umfang durchgehende Schweißnaht, was nur durch das Drehen der Lasereinrichtung bzw. des zu verschweißenden Schlauches erfolgt.

Die WO 2005/063469 offenbart Schlauchstücke, die über sogenannte Manschetten miteinander verbunden werden können. Die Verbindung zwischen Schlauch und Manschette erfolgt über eine Verbindungsschicht, die ein bei 700 bis 2500 nm absorbierendes Material enthält. Hier erfordert der Laserschweißvorgang eine Rotationsbewegung entweder des Lasers bzw. des verschweißenden Schlauches.

Nachteilig bei diesem aus dem Stand der Technik bekannten Laserschweißverfahren ist es also insbesondere, daß die Fügeflächen zwischen den zwei miteinander zu verbindenden Formteilen ringsum vom Laser bestrahlt werden müssen. Dies setzt voraus, daß die miteinander zu verbindende Fläche dem Laser von allen Seiten zugänglich sein muss. Die Geometrie bestimmter Bauteile lässt eine umlaufende Bestrahlung oft nicht zu. Eine Rotation des entsprechenden Bauteils war oftmals geometriebedingt nicht durchführbar, so daß in vielen Fällen eine Laserverschweißung bislang unmöglich war.

Aufgabe der vorliegenden Erfindung war es daher, ein Laserdurchstrahlschweißverfahren bereitzustellen, das den oben erwähnten Nachteilen des Standes der Technik abhilft und insbesondere es ermöglicht, daß eine vollständige umlaufende Verschweißung zweier miteinander zu verbindender Formteile rotations- bzw. drehfrei ermöglicht wird. Ferner ist es Aufgabe der vorliegenden Erfindung eine Vorrichtung bereitzustellen, mit der zwei miteinander zu verbindende Formteile schnell verschweißt werden können, ohne ein Teil der Vorrichtung oder eines der zu verschweißenden Teile drehen zu müssen.

Erfindungsgemäß wird die Aufgabe durch ein Verfahren zum Verschweißen von zwei Fügepartnern nach Anspruch 1 gelöst, bei dem eine Vorrichtung einen Laser und einen Spiegel umfaßt, wobei der Laser, die Fügepartner und der Spiegel so angeordnet werden können, daß der Strahl des Lasers auf die Fügepartner und anschließend auf den Spiegel auftrifft, wobei der Spiegel teilweise die Kontur der inneren Fläche eines Hohlzylinders aufweist. Mit Hilfe dieser Vorrichtung kann die den Absorber enthaltende Schicht gleichmäßiger bestrahlt werden. Es kommt nicht zur Überhitzung punktueller Bereiche des Schlauches und die Schweißnaht wird einheitlicher.

In einer bevorzugten Ausführungsform ist der Strahl des Lasers in einem Strahlabschnitt divergent und der divergente Strahlabschnitt trifft zunächst auf das Schlauchsegment und anschließend auf den Spiegel. Vorteil dieser Ausführungsform ist eine noch gleichmäßigere Bestrahlung der den Absorber enthaltenden Schicht.

Üblicherweise werden die zu verbindenden Fügepartner, das Schlauchsegment und der röhrenförmige Formkörper mit einem Laserstrahl bestrahlt. Zum einen geht ein Großteil der bereitgestellten Laserenergie bei Verschweißen durch Streuungs- und Reflexionseffekte verloren. Alle nicht senkrecht auf die Schlauchoberfläche auftreffenden Strahlen werden abgelenkt, ab einem bestimmten Grenzwinkel werden die Strahlen totalreflektiert und tragen nicht zum Verschweißen der Fügepartner bei. Ferner ist die zu durchdringende laserabsorbierende Materialschicht in den Randbereichen wesentlich größer und trägt somit auch dazu bei, daß den unten liegenden Bereichen weniger Energie zugeführt wird. Durch die Reflexion der Strahlen an dem Spiegel wird Energie genutzt, die ansonsten durch Reflexion oder Transmission verloren gehen würde. Die am Spiegel reflektierten Strahlen können erneut in das Schlauchmaterial eindringen, um für ein Schweißverfahren zur Verfügung stehen.

Die Kontur des Spiegels beschreibt einen Teil einer inneren Oberfläche eines Hohlzylinders. Die Aussparung an einer Seite, die der vollkommen zylindrischen Kontur fehlt, dient als Blende für den einfallenden Laserstrahl. Leichte Abweichungen an der idealen Kontur eines Hohlzylinders sind möglich. Der Querschnitt des Hohlzylinders wäre dann nicht kreisrund, sondern elliptisch.

Ein Laserstrahl ist im Rahmen dieser Erfindung divergent, wenn der Strahlquerschnitt in Strahlrichtung größer wird. Der Vorteil dieser Anordnung liegt darin, daß bei Verwendung der Vorrichtung für ein Schweißverfahren alle Bereiche der den Absorber umfassenden Schicht eines Schlauchsegments gleichmäßig bestrahlt werden können. Dadurch werden Temperaturdifferenzen innerhalb der den Absorber umfassenden Schicht gering gehalten.

Bevorzugt besteht der Spiegel aus Aluminium oder einer Aluminiumlegierung. Es hat sich gezeigt, daß insbesondere Spiegel aus poliertem Aluminium die besten Reflexionseigenschaften aufweist. Spiegel aus Stahl erhitzen sich sehr stark und reflektieren unmerklich. Spiegel aus Messing erhitzen sich auch, reflektieren aber auch einen Teil der Strahlung. Der Reflexionseffekt geht mit zunehmender Abstumpfung der Spiegeloberfläche, die durch Erwärmung entsteht, zurück. Eine gut reflektierende Oberfläche kann aber durch erneutes Polieren wiederhergestellt werden.

Bevorzugt liegt der Durchmesser des Hohlzylinders zwischen 5 und 25 mm, noch bevorzugter bei 20 mm. Der Abstand zwischen Spiegeloberfläche und Oberfläche der Fügepartner sollte nicht zu groß sein, damit nicht zu viel am Spiegel reflektierte Lichtenergie abgestrahlt wird, ohne die Fügepartner zu durchdringen. Ferner können bevorzugt Schläuche mit einem Gesamtdurchmesser von bis zu 13 mm geschweißt werden. Schläuche mit größerem Durchmesser haben gewöhnlich größere Wandstärken. Insbesondere die keinen Absorber enthaltende Schicht ist dicker. Diese Schicht hat ein höheres Wärmeaufnahmevermögen. Mit dieser höheren Wärmeaufnahme wird dem Schweißprozeß Wärme entzogen, so daß es eher zu Schweißnahtfehlern kommen kann. Schlauchgröße und Abstand zwischen Spiegeloberfläche und Oberfläche der Fügepartner sind die begrenzenden Faktoren im Hinblick auf den Durchmesser des Hohlzylinders.

Bevorzugt wird ein Diodenlaser mit einer Wellenlänge von 750 bis 1000 nm, noch bevorzugter mit 808 nm, verwendet. Der Laser kann eine Leistung zwischen 380 und 520 W, bevorzugter eine Leistung von 500 W aufweisen. Mit den Diodenlasern können Laserstrahlen erzeugt werden, die konvergieren und divergieren können. Hierbei handelt es sich um sogenannte "aufgefächerte Diodenarray Laser". Ferner sind die Leistung des Lasers und die Wellenlänge des vom Laser emittierten Lichtes optimal für den Schweißprozeß, so daß keine Schweißnahtfehler entstehen und das polymere Material nicht überhitzt wird. In einem nicht zur Erfindung gehörenden Laserschweißverfahren zur Verbindung eines PVC-freien Schlauchsegments mit einem röhrenförmigen Formkörper ("die Fügepartner") unter Verwendung eines Lasers und eines Spiegels ist die Schlauchwand des Schlauchsegments aus mindestens zwei von einander verschiedenen Materialschichten aufgebaut und eine dieser Schichten enthält zumindest abschnittsweise einen Absorber für Laserstrahlung, S wobei bei dem Verfahren
a) das PVC-freie Schlauchsegment und der röhrenförmige Formkörper bereitgestellt werden,
b) das Schlauchsegment und der röhrenförmige Formkörper formschlüssig ineinandergeschoben werden, so daß sich die Oberflächenbereiche des Schlauchsegments und des röhrenförmigen Formkörpers überlappen,
c) das Schlauchsegment und der röhrenförmige Formkörper mit den sich überlappenden Oberflächenbereichen vor dem Spiegel angeordnet werden,
d) das Schlauchsegment und der röhrenförmige Formkörper mit den sich überlappenden Oberflächenbereichen mit Laserstrahlung bestrahlt werden, so daß die vom Schlauchsegment oder vom röhrenförmigen Formkörper reflektierten oder transmittierten Laserstrahlen von dem Spiegel reflektiert werden und teilweise wieder auf das Schlauchsegment oder den röhrenförmigen Formkörper auftreffen, wobei sich eine Schweißverbindung zwischen dem Schlauchsegment und dem röhrenförmigen Formkörper ausbildet.

Mit diesem Verfahren kann die Bestrahlungsdauer im Vergleich zu Schweißverfahren, bei denen zu verschweißende Fügepartner oder Teile der Vorrichtung bewegt werden müssen, erheblich verkürzt werden. Das durch Reflexion und Transmission an den Fügepartnern abgestrahlte Laserlicht wird am Spiegel reflektiert und steht für ein weiteres Schweißverfahren erneut zur Verfügung, so daß der Laser und/oder die zu verschweißenden Fügepartner nicht bewegt werden müssen. Daher verkürzt sich die Bestrahlungsdauer auf wenige Sekunden. Bevorzugt können die Fügepartner in weniger als 3 Sekunden miteinander verschweißt werden. Somit wird bei dem Verfahren in Schritt d) höchstens 3 Sekunden bestrahlt.

Um optimale Ergebnisse zu erreichen und eine einheitliche Schweißnaht zu bilden, wird bei dem Verfahren ein Spiegel verwendet, der die Kontur der inneren Fläche eines Hohlzylinders hat.

Bei einer hat. Ausführungsform dieses Verfahrens ist der Durchmesser des Laserstrahls beim Auftreffen auf den Schlauchabschnitt geringer als der Durchmesser des zu verschweißenden Schlauchabschnitts. Bevorzugt liegt das Verhältnis des Durchmessers des Laserstrahls zu dem Durchmesser des Schlauchabschnitts zwischen 1 zu 2 und 1 zu 5.

Bei dem dem Verfahren fällt jeglicher Verfahrweg weg, da mit Hilfe des Spiegels die Strahlung gleichmäßig auf die gesamte Fläche abstrahlt. Jeglicher Fahrweg fällt auch dann weg, wenn der Durchmesser des Strahlabschnitts beim Auftreffen auf den Schlauchabschnitt geringer ist als der Durchmesser des Schlauchabschnitts.

Ein weiterer Vorteil des Verfahrens besteht darin, daß sich ein gleichmäßigeres Schweißbild einstellt. Als positiver Nebeneffekt ist der gewonnene Platz zwischen Laseroptik und Schweißstelle zu sehen, wodurch die Fügepartner einfacher in die Halterung eingelegt und herausgenommen werden können.

Für den Fall, daß ein Spiegel eingesetzt wird, der die Kontur der inneren Fläche eines Hohlzylinders aufweist, ist der Abstand zwischen Spiegel und Schlauchwand ringsrum *in etwa* gleich. In dem Fall, daß der Spiegel der Vorrichtung nicht exakt die Kontur eines Hohlzylinders beschreibt, kann der Abstand zwischen Spiegel und Schlauchwand nicht gleich sein. Wenn der Querschnitt des Hohlzylinders elliptisch ist, sind die zu verbindenden Fügepartner zentriert vor dem Spiegel angeordnet.

Die der Erfindung zugrundeliegende Aufgabe wird dadurch gelöst, daß ein Verfahren zum Laserdurchstrahlschweißen zur Verbindung eines PVC-freien Schlauchsegments mit einem röhrenförmigen Formkörper ("die Fügepartner") bereitgestellt wird, wobei die Schlauchwand des Schlauchsegments aus mindestens zwei voneinander verschiedenen Materialschichten aufgebaut ist und eine dieser Schichten zumindest abschnittsweise einen Absorber für Laserstrahlung enthält, umfassend die Schritte:
a) des Bereitstellens des PVC-freien Schlauchsegments und des röhrenförmigen Formkörpers,
b) des formschlüssigen Ineinanderschiebens des Schlauchsegments mit dem röhrenförmigen Formkörper, so daß sich Oberflächenbereiche des Schlauchsegments und des röhrenförmigen Formkörpers überlappen,
c) des Bestrahlens eines ersten im Wesentlichen halbzylindrischen Segments der überlappenden Oberflächenbereiche mit Laserstrahlung einer definierten Wellenlänge,
d) des Abschwächens der Laserstrahlung auf 90-50% der ursprünglichen Energie durch Absorption in der den Absorber enthaltenden Schicht unter Ausbildung einer Schweißverbindung im ersten Segment,
e) des Auftreffens der abgeschwächten Laserstrahlung auf ein zweites im Wesentlichen halbzylindrisches Segment, des überlappenden Oberflächenbereichs das einer Quelle für Laserstrahlung entgegengesetzt angeordnet ist und zum ersten Segment im Wesentlichen spiegelsymmetrisch ist zur Ausbildung einer Schweißverbindung im zweiten Segment.
Das erfindungsgemässe Verfahren ob mit oder ohne Verwendung eines Spiegels, bietet den Vorteil, daß die beiden Fügepartner nur von einer einzigen Seite aus durchstrahlt werden, wobei sich ringsum eine formschlüssige Schweißverbindung der Fügepartner ergibt. Dadurch kann das erfindungsgemässe Verfahren in sich ringsum eine formschlüssige Schweißverbindung der Füge-Bezug auf entweder die Laservorrichtung oder die Fügepartner rotations- bzw. drehungsfrei durchgeführt werden, so daß eine weit weniger aufwendige mechanische Apparatur zur Durchführung des erfindungsgemäßen Verfahrens nötig ist als bei den aus dem Stand der Technik bekannten Verfahren, was somit eine wesentliche Vereinfachung darstellt. Wird ein Verfahren ohne Verwendung eines Spiegels durchgeführt, kann es nötig sein, daß der Laser oder die Fügepartner eine Schiebestrecke durchfahren müssen, um gleichmäßige Schweißnähte zu erzeugen. Vorteil des Verfahrens unter Verwendung der erfindungsgemäßen Vorrichtung ist, daß eine gleichmäßigere Bestrahlung der Fügepartner möglich ist und keine Vorrichtungsteile oder die zu verschweißenden Teile bewegt werden müssen.

Auch bei diesem, die Schritte a) bis e) umfassenden Verfahren kann ein Spiegel zur Reflexion des auf die Fügepartner gerichteten Laserlichts eingesetzt werden. Wird ein Spiegel eingesetzt, umfaßt das Verfahren zusätzlich die Schritte des Auftreffens der abgeschwächten Laserstrahlung auf einen Spiegel, der Reflexion des Laserstrahls und des erneuten Auftreffens des Lichtstrahls auf das Schlauchsegment.

Zur Durchführung der erfindungsgemäßen Verfahren ist es dabei erfindungswesentlich, daß ein mehrschichtig aufgebautes Schlauchsegment bereitgestellt wird, wobei in einer Schicht ein Absorber für Laserstrahlung enthalten sein muß.

Der Begriff "formschlüssig" wird im Vorliegenden so verstanden, daß das Schlauchsegment und der rohrförmige Formkörper spalt- bzw. fugenfrei miteinander überlappen. Das Schlauchsegment übt dabei bevorzugt einen Anpreßdruck auf den rohrförmigen Formkörper aus.

Unter dem Begriff "Schlauchsegment" wird jeder beliebige Abschnitt eines Schlauches beliebiger Länge verstanden, durch den Flüssigkeiten durchgeleitet werden können. Da ein Einsatz als medizinischer Schlauch beispielsweise in Hämodialysesystemen vorgesehen sein kann, ist es nötig, daß der Schlauch insbesondere PVC-frei ausgestaltet ist, da neuere Studien ergeben haben, daß die in PVC (Polyvinylchlorid) verwendeten Weichmacher durch die durch den Schlauch hindurchgeführten biologischen Flüssigkeiten gelöst werden und gesundheitliche Gefahren in einem menschlichen Organismus hervorrufen können.

Die Verschweißbarkeit bzw. die Stabilität der Schweißverbindung wird insbesondere durch die Menge an Absorber für das verwendete Laserlicht in der den Absorber enthaltenden Schicht bestimmt. Erfindungsgemäß enthält eine äußere Oberfläche des Schlauches, d.h. entweder dessen Innenseite oder dessen Außenseite eine funktionalisierte, d.h. den Absorber enthaltende dünne Schicht. Diese Schicht wird mit der darunterliegenden zweiten Schicht typischerweise koextrudiert. Die Schichtdicken der den Absorber enthaltenden Schicht liegen erfindungsgemäß im Bereich von 20 bis 100 µm, so daß sie auch noch im unteren Schichtdickenbereich durch Koextrusion herstellbar sind.

Die Anordnung der den Absorber enthaltenden Schicht entweder als die das Schlauchinnere oder in einer anderen Ausführungsform der Erfindung als die das Schlauchäußere bildenden Oberfläche ermöglicht insbesondere verschiedene geometrische Anordnungen von miteinander zu verschweißenden Formteilen: Zum Beispiel kann in einer bevorzugten Ausführungsform der Erfindung der Schlauch in den röhrenförmigen Formkörper, beispielsweise also in einen Konnektor, gesteckt werden, so daß in diesem Fall die äußere Schicht mit dem Absorber funktionalisiert sein muss, d.h. die Schicht, die mit dem zu verschweißenden zweiten Fügeteil, dem Konnektor, in Berührung kommt.

In einer weiteren vorteilhaften Ausführungsform der Erfindung bildet die funktionalisierte Schicht, d.h. die Schicht, die den Absorber enthält, die Innenseite des Schlauchsegmentes, so daß ein röhrenförmiger Formkörper in den Schlauch eingeführt werden kann.

In weiteren bevorzugten Weiterbildungen der Erfindung enthält nur der Teil der Schicht den Absorber, der mit dem zu verschweißenden Bereich des röhrenförmigen Formkörpers überlappt. Dies ist selbstverständlich bei beiden vorstehend beschriebenen geometrischen Alternativen möglich. Damit kann insbesondere auch der Einsatz von teuren Farbstoffen für die Absorption ermöglicht werden.

Die Konzentration des Absorbers in der ihn enthaltenden Schicht beträgt erfindungsgemäß zwischen 50 bis 300 ppm, bevorzugt 80-200 ppm, und wird so gewählt, daß nur ca. 10-50%, bevorzugt 15-40% des eingesetzten Laserlichts entlang der Durchtrittsweglänge des Laserlichts durch die den Absorber enthaltenden Schicht des ersten im Wesentlichen halbzylindrischen Segments absorbiert werden und somit für die Verschweißung des ersten Teils verwendet werden können. In diesem Zusammenhang versteht es sich, daß der Fachmann Art und Menge des Absorbers auf die Art des eingesetzten Lasers bzw. dessen Wellenlänge abstimmt, um die vorgenannten Werte zu erzielen.

Vorteilhafterweise bestehen die Schichten des erfindungsgemäß einsetzbaren Schlauchsegments aus PVC-freien Materialien. Erfindungsgemäß ist das Material für die den Absorber enthaltende Schicht ausgewählt aus Polyolefinen, Polyethylen, Polypropylen, Polyisopren, Styrol-Olefin Copolymeren, Styrol-Ethylen-Butadien-Styrol Blockcopolymeren (SEBS), Styrol-Ethylen-Propylen-Styrol Blockcopolymeren (SEPS), Styrol-Isopren-Styrol Copolymeren (SIS), Styrol-Ethylen-Butadien Copolymeren (SEB) sowie Mischungen davon.

Die mindestens eine weitere absorberfreie Schicht nimmt insbesondere die Funktion ein, dem Schlauchsegment eine gewisse mechanische Stabilität, Knickresistenz, etc. zu verleihen.

Dafür sind die Materialien für diese Schicht erfindungsgemäß ausgewählt aus den reinen polyolefinischen Materialien Polypropylen, Polyethylen, Polyisopren, sowie Styrol-Ethylen-Butadien-Styrol Blockcopolymere (SEBS), Styrol-Ethylen-Propylen-Styrol Blockcopolymere (SEPS), Styrol-Isopren-Styrol Copolymere (SIS), Styrol-Ethylen-Butadien Copolymere (SEB) sowie Mischungen davon.

In weiteren Ausführungsformen der Erfindung hat das erfindungsgemäß verwendete Schlauchsegment einen inneren Durchmesser von maximal 8 mm und einen äußeren maximalen Durchmesser von 12 mm. Bevorzugte Wandstärken des Schlauchsegments liegen bei 2 mm, in weiteren spezifischen Ausführungsformen wie z. B. bei einer vorgesehenen Verwendung des Schlauches als so genannter Blutsystemschlauch sogar bei bis zu 1,5 mm.
In weiteren vorteilhaften Ausführungsformen kann der Schlauch noch weitere beliebige Abfolgen von Schichten enthalten. Wichtig ist nur, daß diese weiteren Schichten das Laserschweißverfahren nicht beeinträchtigen, so daß diese Schichten im Wesentlichen lasertransparent sein müssen.

Damit können auch komplexe Anforderungen insbesondere hinsichtlich mechanischer Stabilität, Knickresistenz, Elastizität, etc. ausreichend berücksichtigt werden.

Bevorzugt wird im Rahmen der erfindungsgemäßen Verfahren ein so genannter Diodenlaser eingesetzt. Dieser weist eine Wellenlänge bis zu 1000 nm, bevorzugt von 750 bis 900 nm, am meisten bevorzugt von 808 nm auf. Besonders wichtig ist bei der Durchführung der erfindungsgemäßen Verfahren, daß mit oder ohne Anwendung von Druck eine Fügeebene erhalten wird, bei der beide Fügepartner bevorzugt fugenlos miteinander überlappen, so daß die formschlüssige Verbindung durch das durch den Laser erhitzte fließfähige Polymer besonders einfach erhalten wird, so daß eine große Vielfalt bei der Gestaltung der Schweißnahtgeometrie erfindungsgemäß erhalten werden kann. Die am häufigsten verwendete Geometrie beim Fügen von Kunststoffen mit einem Laser, insbesondere mit einem Diodenlaser, ist die Überlappnaht, die auch im vorliegenden Fall bevorzugt eingesetzt wird.

In diesem Zusammenhang ist wichtig, daß die Vielzahl möglicher Lasertypen, die im Rahmen der vorliegenden Erfindung zum Einsatz vorgesehen sind (z. B. Nd:YAG Laser für den Fall, dass das Verfahren ohne Verwendung eines Spiegels durchgeführt wird, oder ein Diodenlaser für das erfindungsgemässe Verfahren, ob Verfahren ohne Verwendung eines Spiegels durchgeführt wird, mit oder ohne Verwendung eines Spiegels, usw.), die dadurch auch unterschiedliche Wellenlängen aufweisen, aufgrund der großen Energiedichte der Laserstrahlung keine Einschränkung hinsichtlich der Absorbersubstanzen bedeuten. Durch die große Energiedichte ist es nicht notwendig, im Absorptionsmaximum der jeweiligen gewählten Absorbersubstanz einzustrahlen. Es genügt, wenn in einem Fenster von circa ± 100 nm um das Maximum der jeweiligen Substanz herum eingestrahlt wird, so daß eine breite Palette von Absorbern für den Einsatz in dem erfindungsgemäßen Verfahren Verwendung findet. Gewöhnlich wird bei dem Verfahren, das die Schritte a) bis e) umfaßt, ein Laser eingesetzt, der weitestgehend parallele Lichtstrahlen emittiert.

In dem erfindungsgemäßen Verfahren, bei dem kein Spiegel für die Reflexion des Laserstrahlen zum Einsatz kommt, beträgt die Abschwächung der Laserstrahlung entlang die Reflexion der Laserstrahlen zum Einsatz kommt, der Durchtrittsweglänge des Laserlichts durch die den Absorber enthaltenden Schicht des ersten im Wesentlichen halbzylindrischen Segments aufgrund des Absorbers ca. 10-50%, bevorzugt 15-40%. Diese Bereiche ermöglichen, daß auch bei längeren Durchtrittswegen an einzelnen Kreissegmenten des Schlauchs noch genug Laserstrahlung das entgegengesetzte Kreissegment des Pumpschlauchs erreicht, so daß auch dort noch eine feste Verschweißung ermöglicht wird.

Für den Fall, daß das Verfahren ohne Verwendung eines Spiegels eingesetzt wird, beträgt die Laserleistung bei einem Diodenlaser üblicherweise ca. 400 Watt. Es versteht sich, daß diese Werte nur Anhaltspunkte für den Fachmann bei der Wahl des geeigneten Lasers sind. Auch bei diesem Verfahren kann als Laser ein "aufgefächerter Diodenarray Laser" mit einer Leistung von circa 400 Watt verwendet werden, der Strahlung auf einen Spot von circa 10 mm Durchmesser fokussiert. Typische Zeiten für die Einwirkung der Laserstrahlung betragen 1-10 s, bevorzugt 2-8 s, ganz besonders bevorzugt 3-7 s, je nach Schlauchdicke. Beispielhafte Zeiten betragen im Falle eines Blutpumpschlauchs mit einem Durchmesser von circa 12 mm 7 Sekunden, bei einem Schlauch mit einem Durchmesser von 6,5 mm circa 3 Sekunden bis zur Ausbildung einer festen Schweißverbindung.

Die Aufgabe der vorliegenden Erfindung wird ferner gelöst durch eine lasergeschweißte Fügeverbindung gemäss Anspruch 11 zwischen einem Schlauchsegment und einem röhrenförmigen Formkörper, die durch die erfindungsgemäßen Verfahren, ob unter Verwendung mit oder ohne Spiegel, hergestellt werden, wobei die Schlauchwand des Schlauchsegments aus mindestens zwei voneinander verschiedenen Materialschichten aufgebaut ist und eine dieser Schichten zumindest abschnittsweise einen Absorber für Laserstrahlung enthält, und wobei das Material für die den Absorber enthaltende Schicht und das Material der absoberfreien Schicht wie vorstehend erläutert ausgewählt sind. Damit sind lasergeschweißte Fügeverbindungen, die ohne besonders großen mechanischen Aufwand hergestellt werden können, insbesondere im medizinischen Bereich einsetzbar, hier insbesondere bei der Förderung von physiologischen Flüssigkeiten, Infusionslösungen oder Blut.

An die zu verschweißenden Schlauch- und Anschlussstücke werden dabei besondere Anforderungen gestellt, so daß die Wahl der entsprechenden Polymere in Bezug auf Transparenz im sichtbaren Wellenlängenbereich, Hitzesterilisierbarkeit und Biokompatibilität beachtet werden müssen, die jedoch aus dem vorstehend aufgeführten Stand der Technik bekannt sind. Daher umfasst die vorliegende Erfindung ebenfalls ein Schlauchsystem, das eine Mehrzahl von derartigen lasergeschweißten Fügeverbindungen zwischen einem Schlauchsegment und einem röhrenförmigen Formkörper, beispielsweise einem Konnektor oder einem weiteren Schlauchsegment umfasst, zur Verwendung beispielsweise in einem Hämodialysekreislauf.

Die Erfindung ist weiter anhand von Ausführungsbeispielen in den nachstehenden Figuren näher erläutert, ohne daß dies als Einschränkung des Erfindungsgedankens verstanden werden soll.

Es zeigen:
- Fig. 1: einen Querschnitt durch eine Verbindung, bei der ein Konnektor auf ein Schlauchsegment geschoben wurde.
- Fig. 2: zeigt eine Verbindung, bei der ein Konnektor in einen Schlauch geschoben wurde.
- Fig. 3: zeigt eine lasergeschweißte Fügeverbindung zwischen einem Konnektor und einen in den Konnektor eingeschobenen Schlauch.
- Fig. 4: zeigt eine Anordnung bestehend aus einem Laser und einem hohlzylindrischen Spiegel.

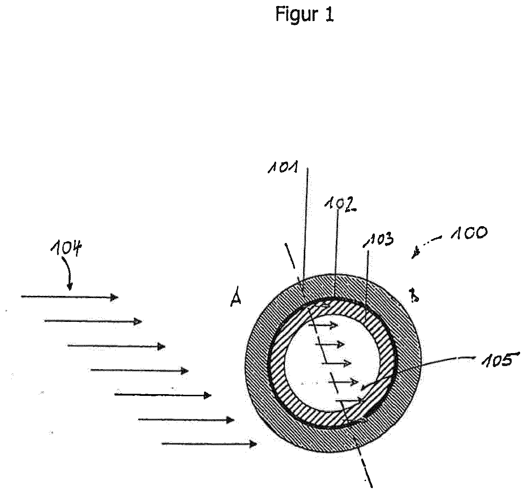

Figur 1 zeigt eine erfindungsgemäße Kombination 100 zwischen einem Konnektor 101 aus Polypropylen und einem Schlauch, der aus einer inneren Schicht 103 sowie aus einer äußeren Schicht 102 besteht, wobei die äußere Schicht 102 ein laserabsorbierendes Material wie beispielsweise einen Farbstoff enthält. Die Schicht 102 weist dabei eine Dicke von ca. 50 µm auf. Als Absorbersubstanz kann beispielsweise Lumogen IR 788 von der Firma BASF verwendet werden, jedoch ist es im Rahmen der vorliegenden Erfindung ebenfalls möglich, daß jeder andere beliebige Absorber, der im Wellenlängenbereich des verwendeten Diodenlasers absorbiert, verwendet wird. Nachdem das Schlauchsegment in den Konnektor 101 geschoben ist, wird eine Laserstrahlung 104 mit einer Wellenlänge von 808 nm über einen Zeitraum von 3-5 Sekunden angelegt. Die Laserstrahlung 104 wird in einem halbzylinderförmigen Bereich, der mit dem Großbuchstaben "A" dargestellt wird, von dem Absorber in der Schicht 102 absorbiert, und schmilzt den Kunststoff auf, so daß eine formschlüssige Verbindung zwischen dem Konnektor 101 aus Polypropylen und dem Schlauch an der äußeren Schicht 102 entsteht. Die Strahlung 104 wird absorbiert und trifft , wie vorstehend detailliert erläutert, um circa 10-50% abgeschwächt, dargestellt durch die Pfeile 105, in das zweite halbkreisförmige Segment B auf, wo sie ebenfalls von dem Absorber in der Schicht 102 absorbiert wird und somit auch im Segment B eine formschlüssige Verbindung durch Aufschmelzen der Schicht 102 zwischen der Schicht 102 des Schlauchsegmentes und dem Konnektor 101 aus Polypropylen hervorruft. Die Reststrahlung wird üblicherweise nicht völlig absorbiert. Aus den Figuren ist auch die zu durchlaufende unterschiedliche Weglänge für die Wellenfront des Laserlichts erkennbar: die Weglänge l₁ ist dabei viel kleiner als die Weglänge l₂, so daß Licht entlang der Weglänge l₁ weniger abgeschwächt wird, also eher im Bereich von 10% Abschwächung liegt, als entlang der Weglänge l₂, wo die Absorption eher bei 50% liegt.

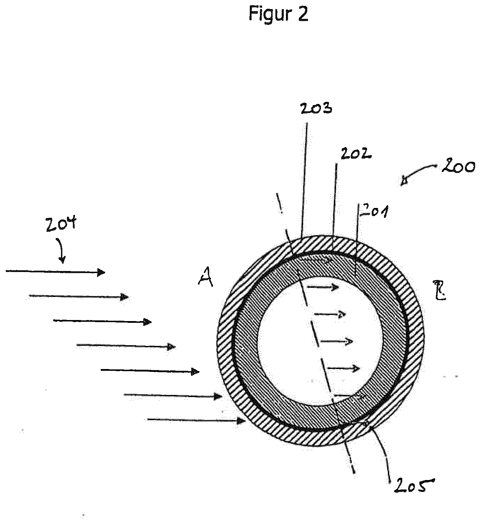

Figur 2 zeigt eine weitere Ausführungsform einer Verbindung 200, die im Rahmen des erfindungsgemäßen Verfahrens eingesetzt werden kann. Dabei wird ein Konnektor 201, beispielsweise aus Polypropylen in ein Schlauchsegment eingeführt, so daß die innenliegende Oberfläche des Schlauchs 202 das laserabsorbierende Material enthält und die nach außen liegende Schicht 203 zur Stabilisierung dient.

Es versteht sich, daß natürlich noch weitere Schichten außen bzw. zwischen den Schichten 202 und 200 angeordnet sein können, solange diese, wie vorstehend schon erwähnt, lasertransparent sind. Die Dicke der Schicht 202 beträgt ebenfalls 50 µm und als absorbierende Substanz wurde ebenfalls Lumogen IR 788 von BASF verwendet.

Die Anordnung 200 wird nun ebenfalls mit Laserstrahlung einer Wellenlänge λ = 808 nm bestrahlt (dargestellt mit Pfeil 204), die im ersten Halbkreis bzw. halbzylinderförmigen Segment A zu ca. 10-50 % absorbiert wird, so daß eine formschlüssige Verbindung zwischen der Oberfläche des Konnektors 201 und der dieStrahlung absorbierenden Schicht 202 entsteht. Die nicht absorbierte Strahlung 205 trifft auf die Schicht 202 im halbzylinderförmigen Segment B 202 auf und führt dort ebenfalls zur Absorption, Erhitzung und Aufschmelzung, so daß eine vollständige Verschweißung im gesamten Umfang des Schlauchsegments mit dem Konnektor erzielt wird.

Eine Rotation des Lasers bzw. der zu verbindenden Fügeelemente 100 bis 200 ist, wie hier gezeigt wurde, mit dem erfindungsgemäßen Verfahren zur Ausbildung einer umlaufenden Schweißnaht somit nicht erforderlich.

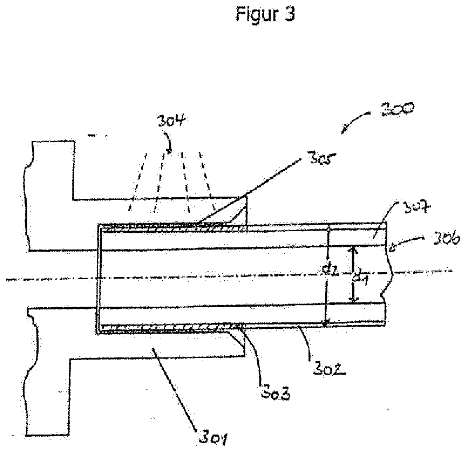

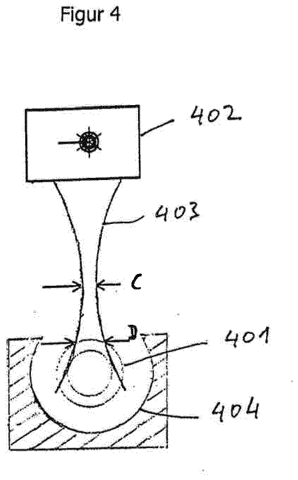

Figur 3 zeigt eine vollständig verschweißte Fügeverbindung 300 zwischen einem Konnektor 301 aus Polypropylen und einem Schlauchsegment 306, das aus einer inneren Schicht 307, die frei von laserabsorbierenden Materialien ist, sowie einer äußeren Schicht 302 die bereichsweise im Abschnitt 303, der mit der Innenseite des Konnektors 301 überlappt, einen Absorber für Laserlicht enthält. Nach Einwirken der Laserstrahlung 304 entsteht, wie in den Figuren 1 und 2 beschrieben, eine formschlüssige umlaufende Schweißnaht 305. Figur 4 zeigt eine nicht zur Erfindung gehörende Vorrichtung 400 zum Verschweißen eines Schlauchsegments 401 bestehend aus einem Laser 402 und einem Spiegel 404. Der Diodenarray Laser emittiert einen Lichtstrahl 403, der in einem ersten Abschnitt konvergiert, in der Ebene C den geringsten Querschnitt aufweist und anschließend divergiert. Der divergente Strahlabschnitt trifft in der Ebene D auf das Schlauchsegment 401 auf. Gestreute und hindurchtretende Laserstrahlen treffen anschließend auf den Spiegel 404 auf, werden dort reflektiert und treffen teilweise wieder auf das Schlauchsegment auf. Durch diese Anordnung wird eine gleichmäßige Bestrahlung der den Absorber umfassenden Schicht gewährleistet, so daß das Schlauchsegment gleichmäßig verschweißt wird.

### Ausführungsbeispiel 1

In einer bevorzugten Ausführung wurde ein PVC-freier Schlauch mit folgendem Aufbau verwendet:

| | |
|---|---|
| Innendurchmesser | 8 mm |
| Außendurchmesser | 12 mm |
| Wandstärke: | 2 mm |

Die den Absorber enthaltende Schicht des Schlauches weist eine Zusammensetzung wie folgt auf:

| | |
|---|---|
| Dicke der Absorberschicht: | 50 µm |
| Zusammensetzung: | 60 % SEBS Tuftec 1221 (Asahi) |
| | 40 % PP-R RD204 CF (Borealis) |
| | 100 ppm Lumogen IR 788 BASF. |

Die absorberfreie Schicht bestand aus einer Mischung aus 70% SEBS Tuftec 1221 (Asahi) und 30% SIS (Hybrar 7125 F, Kuraray).

Das Schlauchsegment mit den beiden Schichten wurde durch Koextrusion hergestellt.

Als zweiter Fügepartner diente ein sogenannter Konnektor aus Polypropylen. Im Ausführungsbeispiel wird das Schlauchsegment wie in Figur 3 beschrieben in den Konnektor aus Polypropylen geschoben, mit dem Unterschied daß die gesamte äußere Schicht den Absorber enthielt.

Die ineinandergesteckten Fügepartner, das Schlauchsegment und der Konnektor wurden mit einem Diodenlaser der Firma Laserline, D-Mühlheim (Diodenlaser LDF 1000-500) mit einer Wellenlänge von 808 nm von einer einzigen Seite aus erfindungsgemäß bestrahlt. Es wurde eine so genannte Durchstrahlschweißung mit Überlappung im Rahmen des erfindungsgemäßen Verfahrens eingesetzt. Eine formschlüssig umlaufende Schweißnaht wurde so erfindungsgemäß ohne die Drehung eines oder beider Fügepartner erhalten.

Die so erhaltene Schweißverbindung erfüllt alle Anforderungen an Dichtigkeit, Zugfestigkeit und Hitzesterilisierbarkeit wie sie im medizinischen Bereich auftreten. Die Zugfestigkeit ("Schlauchausreißfestigkeit") wurde nach DIN 53455 bestimmt und betrug circa 170 N. Im vorliegenden Fall zerriss der Schlauch, während sich die geschweißte Verbindung auch bei dieser Krafteinwirkung nicht löste. Typischerweise betrug die Untergrenze der Zugfestigkeit erfindungemäß verschweißter Verbindungen 120 N und je nach verschweißter Fläche max. 160-170 N, wobei - wie vorstehend ausgeführt - eine genaue Messung der Obergrenze durch das Reißen des jeweiligen Schlauches nicht exakt bestimmt werden konnte.

## Patentansprüche

1. Laserschweißverfahren zur Verbindung eines PVC-freien Schlauchsegments (306) mit einem röhrenförmigen Formkörper (301), wobei die Schlauchwand des Schlauchsegments aus mindestens zwei voneinander verschiedenen Materialschichten (302, 307) aufgebaut ist und eine dieser Schichten zumindest abschnittsweise einen Absorber für Laserstrahlung in einer Konzentration von 50-300 ppm enthält und die Schichtdicke der den Absorber enthaltenden Schicht 20 bis 100 µm beträgt, das Material für die den Absorber enthaltende Schicht ausgewählt ist aus Polyolefinen, Polyethylen, Polypropylen, Polyisopren, Styrol-Olefin Blockcopolymeren, SEBS, SEPS, SIS, SEB und Mischungen davon, und das Material der absorberfreien Schicht ausgewählt ist aus Polyethylen, Polypropylen, Polyisopren, Styrol-Ethylen-Butadien-Styrol Blockcopolymere (SEBS), Styrol-Ethylen-Propylen-Styrol Blockcopolymere (SEPS), Styrol-Isopren-Styrol Copolymere (SIS), Styrol-Ethylen-Butadien Copolymere (SEB) und Mischungen davon, wobei bei dem Verfahren
a) das PVC-freie Schlauchsegment und der röhrenförmige Formkörper bereitgestellt werden,
b) das Schlauchsegment und der röhrenförmige Formkörper formschlüssig ineinandergeschoben werden, so daß sich Oberflächenbereiche des Schlauchsegments und des röhrenförmigen Formkörpers überlappen,
c) ein erstes, im wesentlichen halbzylindrisches Segment der sich überlappenden Oberflächenbereiche mit Laserstrahlung einer definierten Wellenlänge bestrahlt wird,
d) die Laserstrahlung auf 90-50% der ursprünglichen Energie durch Absorption in der den Absorber enthaltenden Schicht unter Ausbildung einer Schweißverbindung im ersten Segment abgeschwächt wird,
e) die abgeschwächte Laserstrahlung auf ein zweites, im Wesentlichen halbzylindrisches Segment des überlappenden Oberflächenbereichs zur Ausbildung einer Schweißverbindung im zweiten Segment auftrifft.

2. Verfahren nach Anspruch 1, **dadurch gekennzeichnet, dass** der Absorber in der die innere Oberfläche des Schlauchsegments bildenden Schicht enthalten ist.

3. Verfahren nach Anspruch 2, **dadurch gekennzeichnet, dass** in Schritt b) der röhrenförmige Formkörper in das Schlauchsegment geschoben wird.

4. Verfahren nach Anspruch 1, **dadurch gekennzeichnet, dass** der Absorber in der die äußere Oberfläche des Schlauchsegments bildenden Schicht enthalten ist.

5. Verfahren nach Anspruch 4, **dadurch gekennzeichnet, dass** in Schritt b) das Schlauchsegment in den röhrenförmigen Formkörper geschoben wird.

6. Verfahren nach einem der vorhergehenden Ansprüche, **dadurch gekennzeichnet, dass** das Schlauchsegment eine Wandstärke von maximal 4 mm aufweist.

7. Verfahren nach einem der vorhergehenden Ansprüche, **dadurch gekennzeichnet, dass** der röhrenförmige Formkörper ein Konnektor oder ein weiteres Schlauchsegment ist.

8. Verfahren nach Anspruch 7, **dadurch gekennzeichnet, dass** der Konnektor im Wesentlichen aus Polypropylen besteht.

9. Verfahren nach einem der vorhergehenden Ansprüche, **dadurch gekennzeichnet, dass** das Schlauchsegment weitere Schichten umfasst.

10. Verfahren nach Anspruch 9, **dadurch gekennzeichnet, dass** die weiteren Schichten für Laserstrahlung durchlässig sind.

11. Lasergeschweißte Fügeverbindung zwischen einem Schlauchsegment und einem röhrenförmigen Formkörper, hergestellt durch das Verfahren gemäß einem der vorhergehenden Ansprüche, wobei die Schlauchwand des Schlauchsegments aus mindestens zwei voneinander verschiedenen Materialschichten aufgebaut ist und eine dieser Schichten zumindest abschnittsweise einen Absorber für Laserstrahlung enthält, wobei das Material für die den Absorber enthaltende Schicht ausgewählt ist aus Polyolefinen, Polyethylen, Polypropylen, Polyisopren, Styrol-Olefin Blockcopolymeren, SEBS, SEPS, SIS, SEB und Mischungen davon, und das Material der absorberfreien Schicht ausgewählt ist aus Polyethylen, Polypropylen, Polyisopren, Styrol-Ethylen-Butadien-Styrol Blockcopolymere (SEBS), Styrol-Ethylen-Propylen-Styrol Blockcopolymere (SEPS), Styrol-Isopren-Styrol Copolymere (SIS), Styrol-Ethylen-Butadien Copolymere (SEB) und Mischungen davon.

12. Schlauchsystem enthaltend eine Mehrzahl von lasergeschweißten Fügeverbindungen zwischen einem Schlauchsegment und einem röhrenförmigen Formkörper nach Anspruch 11, zur Verwendung in einem Hämodialysekreislauf.

## Claims

1. Laser welding method for connecting a PVC-free tube segment (306) with a tubular shaped body (301), wherein the tube wall of the tube segment is composed of at least two material layers (302, 307) different from each other and one of these layers contains at least in parts an absorber for laser radiation in a concentration of 50-300 ppm and the layer thickness of the absorber-containing layer is 20 to 100 µm, the material for the absorber-containing layer is selected from polyolefins, polyethylene, polypropylene, polyisoprene, styrene-olefin block copolymers, SEBS, SEPS, SIS, SEB and mixtures thereof, and the material of the absorber-free layer is selected from polyethylene, polypropylene, polyisoprene, styrene-ethylene-butadiene-styrene block copolymers (SEBS), styrene-ethylene-propylene-styrene block copolymers (SEPS), styrene-isoprene-styrene copolymers (SIS), styrene-ethylene-butadiene copolymers (SEB) and mixtures thereof, wherein in the method
a) the PVC-free tube segment and the tubular shaped body are provided,
b) the tube segment and the tubular shaped body are pushed into one another in a form-fitting manner, so that surface regions of the tube segment and the tubular shaped body overlap,
c) a first, substantially semicylindrical segment of the overlapping surface areas is irradiated with laser radiation of a defined wavelength,
d) the laser radiation is attenuated to 90-50% of the original energy by absorption in the absorber-containing layer to form a welded joint in the first segment,
e) the attenuated laser radiation hits a second, substantially semi-cylindrical segment of the overlapping surface area to form a welded joint in the second segment.

2. The method according to claim 1, **characterized in that** the absorber is contained in the inner surface of the tube segment forming layer.

3. The method according to claim 2, **characterized in that** in step b) the tubular shaped body is pushed into the tube segment.

4. The method according to claim 1, **characterized in that** the absorber is contained in the outer surface of the tube segment forming layer.

5. The method according to claim 4, **characterized in that** in step b) the tube segment is pushed into the tubular shaped body.

6. The method according to any one of the preceding claims, **characterized in that** the tube segment has a wall thickness of at most 4 mm.

7. The method according to any one of the preceding claims, **characterized in that** the tubular shaped body is a connector or another tube segment.

8. The method according to claim 7, **characterized in that** the connector consists essentially of polypropylene.

9. The method according to any one of the preceding claims, **characterized in that** the tube segment comprises further layers.

10. The method according to claim 9, **characterized in that** the further layers are transparent to laser radiation.

11. A laser-welded joint connection between a tube segment and a tubular shaped body, produced by the method according to one of the preceding claims, wherein the tube wall of the tube segment is composed of at least two material layers different from each other and one of these layers contains at least in parts an absorber for laser radiation, wherein the material for the absorber-containing layer is selected from polyolefins, polyethylene, polypropylene, polyisoprene, styrene-olefin block copolymers, SEBS, SEPS, SIS, SEB and mixtures thereof, and the material of the absorber-free layer is selected from polyethylene, polypropylene, polyisoprene, styrene-ethylene-butadiene-styrene block copolymers (SEBS), styrene-ethylene-propylene-styrene block copolymers (SEPS), styrene-isoprene-styrene copolymers (SIS), styrene-ethylene-butadiene copolymers (SEB) and mixtures thereof from.

12. A tube system comprising a plurality of laser-welded joint connections between a tube segment and a tubular shaped body according to claim 11 for use in a hemodialysis circuit.

## Revendications

1. Procédé de soudage au laser pour raccorder un segment de tube sans PVC (306) à un corps de forme tubulaire (301), dans lequel la paroi de tube du segment de tube est composée d'au moins deux couches de matériau (302, 307) différentes l'une à l'autre et l'une de ces couches contient au moins en partie un absorbeur pour le rayonnement de laser à une concentration de 50-300 ppm et l'épaisseur de la couche contenant l'absorbeur est de 20 à 100 µm, le matériau pour la couche contenant l'absorbant étant choisi parmi les polyoléfines, le polyéthylène, le polypropylène, le polyisoprène, les copolymères blocs styrène-oléfine, SEBS, SEPS, SIS, SEB et leurs mélanges, et le matériau de la couche sans absorbant étant choisi parmi le polyéthylène, le polypropylène, le polyisoprène, les copolymères blocs styrène-éthylène-butadiène-styrène (SEBS), les copolymères blocx styrène-éthylène-propylène-styrène (SEPS), les copolymères styrène-isoprène-styrène (SIS), les copolymères styrène-éthylène-butadiène (SEB) et leurs mélanges, dans lequel pendant le procédé
a) le segment de tube sans PVC et le corps de forme tubulaire sont prévus,
b) le segment de tube et le corps de forme tubulaire sont poussés l'un dans l'autre d'une manière ajustée, de sorte que les régions de surface du segment de tube et du corps de forme tubulaire se chevauchent,
c) on irradie un premier segment pratiquement semi-cylindrique des zones de surface en chevauchement avec un rayonnement de laser d'une longueur d'onde définie,
d) le rayonnement de laser est atténué à 90-50% de l'énergie d'origine par absorption dans la couche contenant l'absorbeur en formant un joint soudé dans le premier segment,
e) le rayonnement de laser atténué frappe un second segment pratiquement semi-cylindrique de la zone de surface en chevauchement pour former un joint soudé dans le second segment.

2. Procédé selon la revendication 1, **caractérisé en ce que** l'absorbeur est contenu dans la surface interne de la couche formant un segment de tube.

3. Procédé selon la revendication 2, **caractérisé en ce que** dans l'étape b) le corps de forme tubulaire est poussé dans le segment de tube.

4. Procédé selon la revendication 1, **caractérisé en ce que** l'absorbeur est contenu dans la surface externe de la couche formant un segment de tube.

5. Procédé selon la revendication 4, **caractérisé en ce qu'**à l'étape b) le segment de tube est poussé dans le corps de forme tubulaire.

6. Procédé selon l'une des revendications précédentes, **caractérisé en ce que** le segment de tube a une épaisseur de paroi d'au plus 4 mm.

7. Procédé selon l'une des revendications précédentes, **caractérisé en ce que** le corps de forme tubulaire est un connecteur ou un autre segment de tube.

8. Procédé selon la revendication 7, **caractérisé en ce que** le connecteur est constitué essentiellement de polypropylène.

9. Procédé selon l'une des revendications précédentes, **caractérisé en ce que** le segment de tube comprend d'autres couches.

10. Procédé selon la revendication 9, **caractérisé en ce que** les autres couches sont transparentes au rayonnement de laser.

11. Raccord de jonction soudé au laser entre un segment de tube et un corps de forme tubulaire, produit par le procédé selon l'une des revendications précédentes, dans lequel la paroi du tube du segment de tube est composée d'au moins deux couches de matériau différentes l'une de l'autre et l'une de ces couches contient au moins en partie un absorbeur pour rayonnement de laser, le matériau pour la couche contenant l'absorbant étant choisi parmi les polyoléfines, le polyéthylène, le polypropylène, le polyisoprène, les copolymères blocs styrène-oléfine, SEBS, SEPS, SIS, SEB et leurs mélanges, et le matériau de la couche sans absorbant étant choisi parmi le polyéthylène, le polypropylène, le polyisoprène, les copolymères blocs styrène-éthylène-butadiène-styrène (SEBS), les copolymères blocs styrène-éthylène-propylène-styrène (SEPS), le styrène-isoprène copolymères de styrène-éthylène-butadiène (SEB) et leurs mélanges.

12. Système de tubes comprenant une pluralité de raccords de jonction soudés au laser entre un segment de tube et un corps de forme tubulaire selon la revendication 11 destiné à être utilisé dans un circuit d'hémodialyse.
